Europäisches Patentamt

European Patent Office

Office européen des brevets

(11) Publication number: **0 085 261**
**B1**

(12) **EUROPEAN PATENT SPECIFICATION**

(45) Date of publication of patent specification: **18.09.85**

(51) Int. Cl.⁴: **G 01 N 33/72**

(21) Application number: **82307021.4**

(22) Date of filing: **31.12.82**

(54) **Home diagnostic aid and method for determining the presence of occult blood for use by the general public.**

(30) Priority: **05.01.82 US 337143**

(43) Date of publication of application:
**10.08.83 Bulletin 83/32**

(45) Publication of the grant of the patent:
**18.09.85 Bulletin 85/38**

(84) Designated Contracting States:
**BE DE FR GB NL**

(56) References cited:
**DE-A-2 652 545**
**DE-A-2 716 061**
**US-A-3 996 006**
**US-A-4 175 923**

(73) Proprietor: **HEMATEC CORPORATION**
**Cable, Howse and Coyadd**
**11201 S.E. 8th Suite 163**
**Bellevue Washington 98004 (US)**

(72) Inventor: **Wells, Henry J.**
**1145 Norwood Drive**
**Beaumont TX 77706 (US)**

(74) Representative: **Jones, Michael Raymond et al**
**HASELTINE LAKE & CO. Hazlitt House 28**
**Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a home diagnostic aid and method for determining the presence of hemoglobin in an aqueous medium, and more particularly, to a diagnostic aid and method for determining the presence of occult blood in fecal matter present in an aqueous medium, and most particularly, to a diagnostic aid and method for determining the presence of occult blood in fecal matter with an easily used and comprehended test procedure that employs a single diagnostic aid in only a single manipulative step and eliminates the necessity of handling the fecal matter prior to or during the test procedure and the handling of the test materials once the test is completed.

Over 100,000 persons in the United States are affected by cancer of the colon and rectum per year, occurring equally in both the male and female. When the number of colorectal cancers occurring each year is combined with the number of cancers occurring in other digestive organs, including the esophagus and stomach, such cancers of the digestive system account for more occurrences of cancer than any other single form of the disease. Contrary to many other forms of cancer, early diagnosis and treatment of digestive tract cancer does result in a cure rate of 80% to 90% of those persons affected by the disease. If, however, the disease is not detected until the later stages, the cure rate can drop drastically to 25% or less. Thus, early detection of the disease is critical to successful treatment of digestive tract cancer.

Most, but not all, cancers of the digestive tract bleed to a certain extent. This blood is deposited on and in fecal matter excreted from the digestive system. The presence of blood in fecal matter is not normally detected, however, until gross bleeding, that is, blood visible to the naked eye, occurs. Most advanced cancers cause gross bleeding.

It is known that digestive tract cancers in the early stages also tend to bleed, giving rise to occult (hidden) blood in the fecal matter. Test equipment and test procedures have been developed for use by physicians in testing for the presence of occult blood in fecal matter. One of the most successful tests is manufactured and sold by Smith Kline Diagnostics of Sunnyvale, California, under the trademark "Hemoccult". The package for the "Hemoccult" test is disclosed in U.S. Patent No. 3,996,006 issued to J. F. Pagano. Briefly, the Pagano test employs an absorbent white paper impregnated with a guaiac reagent and encased in a special test slide having openable flaps on both sides of the test slide. To use the Pagano test slide, one must obtain a sample of fecal matter, smear it onto the guaiac-impregnated paper by opening the panel on one side of the test slide, and thereafter close the panel. A panel on the opposite side of the test slide is then opened and a developing agent, which is a stabilized solution of hydrogen peroxide and denatured alcohol, is applied to the guaiac-impregnated paper. If occult blood is present in the fecal matter smeared on the opposite side of the paper, the product of the guaiac reaction will appear as a blue substance against the white paper background, providing a positive indication of the presence of blood in the fecal matter.

Although the Pagano test is excellent for use by physicians in their offices and by diagnostic laboratories, it is not the type of test that is readily adaptable for use by the ordinary person because of his adverse reaction to handling fecal matter and because of his lack of skill in interpreting the results. As stated above, the Pagano test requires that a specimen of fecal matter be obtained. Normally, a specimen is obtained by procuring a sample on the end of a spatula or a wooden depressor, which is then used to smear the specimen on the paper in the Pagano test slide. Once the sample is obtained and the test procedure completed, both the test slide and the spatula or depressor must be disposed of. Disposal of the used materials can and does present a physical problem to, if not an adverse psychological reaction from, the ordinary person. Thus, the ordinary person is not likely to use the Pagano test because of its uncleanly nature (at least apparently so to the ordinary person) and because of the disposal problems associated with the used test slide and spatula or depressor. Additionally, the ordinary person does not necessarily have the skill required to analyze, and thus form accurate conclusions from, the test results.

As an alternative, the ordinary person can initiate the Pagano test in his home and then forward the test slide to his physician or a laboratory for addition of the developing agent and analysis of the test. This procedure, however, requires cold storage of the test slide and specimen if there is a significant time lapse before the test can be completed. Certainly the ordinary person does not wish to store a fecal specimen in his household refrigerator, normally the only cold storage available to him, until he can present the specimen to his physician or an appropriate laboratory. Thus, the general public is not likely to follow or comply with this alternative.

Another test for occult blood is suggested by D. E. Fonner in U.S. Patent No. 2,838,377. The Fonner test, as disclosed, can be effected in a toilet bowl containing fecal matter. The basic test reagents employed by Fonner are o-tolidine and benzidine. These reagents in the presence of blood and other reactants produce a dye visible to the naked eye. Although the Fonner test appears to be a solution to the problem of finding a viable home test for occult blood, it has not met with success for two reasons. First, the above-listed reagents are in themselves known to cause cancer and thus are not suitable for general public distribution. Additionally, the Fonner reagents have a relatively high rate of providing false indications of the presence of occult blood.

Thus, to date, the use of the Pagano test, the Fonner test, and other similar tests has been limited primarily to physicians and diagnostic laboratories. Although this limitation might not at first glance present a significant problem, it does limit the early detection of digestive tract cancers, primarily because patients will not see a physician until other symptoms of digestive tract cancers, such as gross bleeding, manifest themselves. Thus, early detection of cancer of the digestive tract still does not occur with the great majority of patients who contract the disease.

Until the advent of the present invention, the most viable method for testing for occult blood in the home is that disclosed by W. G. Friend in U.S. Patent No. 4,175,923, assigned to Hematec Corporation of Bellevue, Washington. The Friend test again uses the reliable and time-proven test reagent guaiac. In accordance with the preferred embodiment disclosed by Friend, guaiac is impregnated on an absorbent substrate such as an absorbent laboratory filter paper. A developing solution, comprising an alcohol and a peroxide, is applied to the guaiac-containing absorbent substrate. The activated test substrate is then deposited in a toilet bowl, for example, containing feces. If occult blood is present, the guaiac is oxidized to a blue dye that is visible against the absorbent substrate.

While the Friend test overcomes some of the drawbacks of the Pagano test and the Fonner test, the Friend test still has its disadvantages. First, the alcohol-guaiac solution is highly flammable, presenting a potential hazard to a user in the bathroom, a common smoking area. Secondly, the addition of the solution to the test substrate in both the Pagano and Friend tests will almost always leave a brown or blue green ring on the substrate unless the solution is evenly distributed over the entire substrate. This ring can easily be misinterpreted by the inexperienced person as a positive test result. As a consequence, it is desirable to eliminate these problems.

The document DE—A—2652545 discloses a test piece comprising an adsorbent or absorbent carrier having guaiac absorbed thereon or absorbed therein, and an oxidizing agent in contact with said carrier.

According to the present invention, there is provided a substantially dry diagnostic aid, suitable for use in a one-step diagnosis, comprising an adsorbent or absorbent carrier onto or into which a solution of guaiac in a solvent therefor has been adsorbed or absorbed, and, in contact with the carrier, an oxidizing agent capable of oxidizing the guaiac to a blue dye in the presence of water and hemoglobin, the oxidizing agent, in the absence of water, being substantially non-reactive with the guaiac when adsorbed onto or absorbed into the carrier, and wherein there is provided a water-pervious vehicle holding the carrier and the oxidizing agent; characterised in that the carrier is in particulate form, is chosen from a silica based material, calcium phosphate and mixtures thereof, and holds the guaiac readily available to react, in the presence of hemoglobin and the oxidizing agent in an aqueous environment, to yield the blue dye.

The present invention also provides a method of determining the presence of hemoglobin in an aqueous medium, characterised in that it comprises contacting the aqueous medium with a diagnostic aid of the invention, and determining whether a portion of the carrier of said diagnostic aid is dyed blue.

The term "adsorbent" (and related terms) used hereinbelow means "adsorbent and/or absorbent".

The present invention achieves the desired result by providing a diagnostic aid employing the guaiac reaction that is used by performing the single manipulative step of merely depositing a single package in an aqueous medium to be tested for the presence of occult blood. Once the package is in the aqueous medium, it is observed for the characteristic blue dye reaction to determine whether occult blood is present in the aqueous medium. All of the necessary components for causing guaiac to react in an aqueous medium are present in a single novel package in a dry form that does not require the addition of a liquid developer by the user, as has been required by all prior tests employing guaiac since the guaiac reaction was discovered over one hundred years ago.

The carrier, and preferably the oxidizing agent is a dry, particulate material.

A diagnostic aid prepared in accordance with the present invention is inexpensive, clean, nontoxic, non-carcinogenic, and is otherwise safe to handle. The aid has a long shelf life and is very simple to use. Since a positive reaction by the aid yields a bright blue dye that is highly visible against the background of the carrier and/or the oxidizing agent, the test is easily interpreted with little likelihood of error.

The diagnostic aid manufactured in accordance with the present invention places all of the components necessary to yield a positive guaiac reaction in the presence of hemoglobin (one that produces a blue dye) into a single package containing only dry ingredients. To use the diagnostic aid, the single package need only be deposited in an aqueous medium containing the material to be tested for the presence of occult blood, for example, a toilet bowl containing feces. The carrier is, and the oxidizing agent may be, particulate in form and are preferably white so that the blue dye produced by the guaiac reaction in the presence of hemoglobin can be readily observed by the naked eye. As will be understood by reading further, the single package constituting the reactive test system can also take forms other than a particulate mass.

The primary purpose of the carrier is to receive and hold guaiac in a form in which it will be readily available to react in the presence of hemoglobin and a suitable oxidizing agent. Since guaiac alone will not readily react with an oxidizing agent and hemoglobin in an aqueous environment, the guaiac is first dissolved in a solvent therefor and in accordance with the present invention is adsorbed onto the particulate carrier.

A variety of adsorbent particulate carriers can be employed to adsorb and retain the guaiac in the diagnostic aid. The principal requirement for the carrier is that it must be capable of adsorbing a solution of guaiac and, once the solution is adsorbed, remain in an essentially dry state. The carrier is in particulate form so that a guaiac solution can easily be combined and homogeneously mixed throughout the carrier. Silica-based materials including silica and silica gel, and/or calcium phosphate are employed as particulate adsorbent carriers. A presently preferred carrier is the micron-sized silica gel marketed under the trade name "syloid" by the Davison Chemical Division of W. R. Grace & Company of Baltimore, Maryland.

Heretofore, it was thought that only water-miscible solvents could be employed to dissolve guaiac in order to effectively obtain a reproducible guaiac reaction with hemoglobin in an aqueous medium. However, both water-miscible and immiscible solvents can be employed when the guaiac solution is adsorbed onto a carrier and oxidized in the presence of hemoglobin in an aqueous medium. The preferred solvents, however, are the water-miscible solvents such as alkanols and alkenols having from one to six carbon atoms, and particularly the lower aliphatic alcohols. The most preferred alcohols include methanol and particularly isopropyl alcohol. Among the water-immiscible solvents that have been found efficacious are chloroform and benzene.

One of the primary objectives of the present invention is to produce a dry diagnostic aid that is comprised in a single composition or package. Therefore, since the carrier on which the guaiac solution has been adsorbed is dry and particulate, it is preferred that the oxidizing agent employed in the system also be in dry form. The oxidizing agent is preferably of the type that will yield hydrogen peroxide in the presence of water. The hydrogen peroxide, of course, is the oxidant that promotes the reaction between guaiac and hemoglobin to produce the blue reaction product. The oxidizing agent must be capable of being combined with the guaiac-containing carrier to form a single composition or package that will readily react in an aqueous system in the presence of blood. Additionally, the oxidizing agent must be relatively inert with respect to the guaiac in the absence of water. Yet, the oxidizing agent must readily yield its hydrogen peroxide in the presence of water so that the guaiac reaction will take place in a relatively short time after the diagnostic aid has been inserted into the aqueous reaction medium.

Oxidizing agents that will produce hydrogen peroxide in the presence of water and that are dry, stable particulate compositions include monopersulfate compounds, peroxide compounds such as cumene hydroperoxide, tertiary butyl peroxide, barium, strontium and magnesium peroxide, benzoyl peroxide, and potassium perborate. The presently preferred oxidizing agent is potassium monopersulfate, a white, granular, free-flowing powder. This monopersulfate compound is commercially available as a triple salt comprised of two mols of potassium monopersulfate, one mol of potassium hydrogen sulfate, and one mol of potassium sulfate under the trade name "Oxone" from E. I. DuPont de Nemours & Company, Inc. of Wilmington, Delaware.

It has also been found that liquid oxidizing agents can be adsorbed onto a suitable carrier, for example, a silica gel such as the Syloid composition identified above. Additionally, some of the soluble peroxide compounds and relatives thereof can also be dissolved and then adsorbed onto a similar silica gel. After the liquid or dissolved oxidizing agent is adsorbed onto a suitable carrier, that carrier is then contacted with a guaiac-containing carrier to form a single composition. For example, hydrogen peroxide can be adsorbed onto a silica gel such as Syloid. Additionally, the peroxide compounds such as cumene hydroperoxide and tertiary butyl hydroperoxide can be dissolved in isopropyl alcohol or adsorbed directly onto a silica gel carrier. Although these latter oxidizing agents are effective to produce a noticeable and accurate guaiac reaction, none functions as well as the monopersulfate compound identified above.

In practice, guaiac can be combined with a solvent such as isopropyl alcohol in an amount from one to five percent by weight guaiac based on the total solution. It is preferred that from one to about three percent by weight of guaiac be employed in the solvent. The most preferred composition constitutes three percent by weight guaiac in isopropanol. The guaiac solution is combined with a silica gel carrier by slowly adding it to the silica gel while stirring the silica gel. It is preferred that the guaiac solution be combined with a silica gel carrier such as Syloid in proportions of from 1 ml of solution to 6 grams of carrier to 15 ml of solution to 1 gram of carrier. It is most preferable, however, that on the order of one ml of solution per gram of carrier be employed so that the test is not overly sensitive.

When a particulate oxidizing agent such as a monopersulfate compound is employed, the guaiac-containing carrier is combined with an oxidizing agent in a weight range of from one part guaiac-containing carrier to three parts oxidizing agent, to three parts by weight guaiac-containing carrier to one part oxidizing agent. A one-to-one proportion is the most preferred at the present time as it yields the most vivid guaiac reaction in the presence of hemoglobin.

In order to yield an effective diagnostic aid, the guaiac-containing carrier as well as the oxidizing agent must be placed in intimate contact with each other and maintained in contact when placed in an aqueous environment to determine whether occult blood is present in that environment. A variety of packaging techniques may be employed to maintain contact between the guaiac-containing carrier and the oxidizing agent. For example, nonwoven liquid-pervious mats such as filter paper can be employed. The guaiac-containing carrier and oxidizing agent can be thoroughly intermixed and placed on or can be layered on a first liquid-pervious mat followed by an overlay of the second mat to form a water-pervious sandwich. The periphery of the sandwich can be adhesively secured to retain the carrier and oxidizing agent within the sandwich. When the sandwich is placed in a water environment, water plus any occult blood passes through the filter paper and comes into contact with the oxidizing composition and guaiac reagent. If occult blood is present, the oxidation reaction produces the characteristic blue reaction product. One particularly efficacious filter paper has been found to be one available from The Dexter Corporation, Windsor Locks, Connecticut. This material is very similar to that employed in a conventional tea bag.

As an alternative to the foregoing packaging technique, the oxidizing agent can be dissolved in a suitable solvent and impregnated into a suitable liquid-pervious substrate, such as the filter paper described above. The solvent can then be evaporated leaving the dry oxidizing agent in the substrate. For example, an oxidizing agent such as potassium monopersulfate can be dissolved in water. The resulting solution can then be absorbed by a filter paper substrate. Thereafter the filter paper can be air dried, evaporating the solvent and leaving the oxidizing agent in the paper. A guaiac-containing carrier can then be placed on the substrate followed by an overlay of a liquid-pervious mat to provide all of the required test reactants in a single package.

In addition to the foregoing packaging techniques, it is possible to adhesively secure the guaiac-containing carrier to one side, for example, of a liquid-pervious filter paper while the oxidizing agent is adhesively secured to the opposing side of the paper. Additionally, the materials can be compressed into tablet form with a suitable binder. It is also possible to include an effervescing material in tablet or layered form that will react upon contact with water to produce a gas, which in turn will tend to mix the contents of the aqueous medium and enhance the guaiac reaction if occult blood is present.

As will be readily recognized by one of ordinary skill, the present invention represents a significant advance over prior art diagnostic aids employing the guaiac reaction. All that need be done with the single composition system, which includes the guaiac-containing carrier and oxidizing agent, is to place the composition in an aqueous medium, such as a toilet bowl, the contents of which are to be tested for occult blood, and to observe the composition for the characteristic color change. The diagnostic aid and method for determining the presence of occult blood in accordance with the present invention do not require the handling of feces samples or the application of the same to a paper substrate.

Neither does the present invention require the handling or application of a liquid developing solution to a substrate or carrier. Instead, in accordance with the present invention, a single, dry package containing all of the required test reactants is placed in contact with the aqueous medium and thereafter observed for the blue dye that results from the guaiac oxidation reaction. Additionally, a positive reaction with the diagnostic aid prepared in accordance with the present invention yields a bright, vivid blue dye that is very easy to interpret, even by the inexperienced home user.

Although the present invention has been described in terms of a preferred embodiment and various alternatives thereto, one of ordinary skill after reading the foregoing specification will be able to effect various changes, substitutions of equivalents, and other alterations without departing from the invention. For example, a small amount of hemoglobin can be incorporated into the diagnostic aid as a comparison control to assist the ordinary person in correctly interpreting a positive test result. The hemoglobin can be impregnated into a designated location on the filter paper in which reactant compositions are packaged. The user of the aid, of course, would be instructed of the location of the control so that the blue dye produced by the presence of hemoglobin at the designated location would not be mistaken for a positive test, but rather would be used only as an interpretation aid.

## Claims

1. A substantially dry diagnostic aid, suitable for use in a one-step diagnosis, comprising an adsorbent or absorbent carrier onto or into which a solution of guaiac in a solvent therefor has been adsorbed or absorbed, and, in contact with the carrier, an oxidising agent capable of oxidising the guaiac to a blue dye in the presence of water and hemoglobin, the oxidising agent, in the absence of water, being substantially non-reactive with the guaiac when adsorbed onto or absorbed into the carrier, wherein there is provided a water-pervious vehicle holding the carrier and oxidising agent; characterised in that the carrier is in particulate form, is chosen from a silica based material, calcium phosphate and mixtures thereof, and holds the guaiac readily available to react, in the presence of hemoglobin and the oxidising agent in an aqueous environment, to yield the blue dye.

2. A diagnostic aid as claimed in Claim 1, wherein said vehicle comprises first and second sheets of porous paper between which are located said carrier and said oxidising agent.

3. A diagnostic aid as claimed in Claim 1 or 2, wherein said solvent is a lower aliphatic alcohol, chloroform, benzene, or a mixture thereof.

4. A diagnostic aid as claimed in Claim 3, wherein said solvent is an alkanol or alkenol having from one to six carbon atoms.

5. A diagnostic aid as claimed in Claim 4, wherein said solvent comprises methanol, isopropanol, or a mixture thereof.

6. A diagnostic aid as claimed in any of Claims 1 to 5, wherein said guaiac is present in said solution in an amount of from one percent to five percent by weight of solution before being adsorbed onto or absorbed into said carrier.

7. A diagnostic aid as claimed in Claim 6, wherein said guaiac is present in said solution in an amount of from one percent to three percent by weight of solution.

8. A diagnostic aid as claimed in any of Claims 1 to 7, wherein the weight ratio of said oxidising agent to said carrier is from about 1:3 to about 3:1.

9. A diagnostic aid as claimed in any of Claims 1 to 8, wherein said oxidising agent is particulate.

10. A diagnostic aid as claimed in Claim 9, wherein said oxidising agent is potassium persulfate or other monopersulfate compound, cumene hydroperoxide, tertiary butyl hydroperoxide, or a mixture thereof.

11. A diagnostic aid as claimed in Claim 9, wherein said oxidising agent is a triple salt of potassium monopersulfate, potassium hydrogen sulfate and potassium sulfate.

12. A diagnostic aid as claimed in any preceding claim, wherein said carrier and said oxidising agent are both particulate, and are in admixture.

13. A diagnostic aid as claimed in any of Claims 1 to 8, wherein said oxidising agent is in liquid form, and is adsorbed onto or absorbed into a carrier.

14. A diagnostic aid as claimed in Claim 13, wherein said oxidising agent and said solution of guaiac are adsorbed onto or absorbed into a common carrier.

15. A diagnostic aid as claimed in any of Claims 1 to 8, wherein said oxidising agent is in solution in a solvent therefor, said oxidising agent in solution being adsorbed onto or absorbed into a carrier.

16. A diagnostic aid as claimed in Claim 15, wherein said oxidising agent is cumene hydroperoxide, tertiary butyl hydroperoxide, barium peroxide, strontium peroxide magnesium peroxide, or a mixture thereof.

17. A diagnostic aid as claimed in any of Claims 13 to 15, wherein said oxidising agent is hydrogen peroxide.

18. A method of determining the presence of hemoglobin in an aqueous medium, characterised in that it comprises contacting the aqueous medium with a diagnostic aid as claimed in any of Claims 1 to 17 and determining whether a portion of the carrier of said diagnostic aid is dyed blue.

**Revendications**

1. Un agent auxiliaire de diagnostic sensiblement sec convenant à l'utilisation à un diagnostic en une seule étape qui comprend un support adsorbant ou absorbant sur lequel ou dans lequel une solution de gaiac dans son solvant a été adsorbée ou absorbée et, au contact du support, un agent d'oxydation capable d'oxyder le gaiac en un colorant bleu en la présence d'eau et d'hémoglobines, l'agent d'oxydation étant, en l'absence d'eau, sensiblement non réactif avec le gaiac lorsqu'il est adsorbé sur le support ou absorbé dans celui-ci, dans lequel est disposé un véhicule perméable à l'eau qui maintient le support et l'agent d'oxydation, caractérisé en ce que le support, sous une forme particulière, est choisi entre un matériau à base de silice, le phosphate de calcium et leurs mélanges, qu'il garde le gaiac prêt à réagir en présence d'hémoglobines et de l'agent d'oxydation dans un environnement aqueux, pour produire le colorant bleu.

2. Un agent auxiliaire de diagnostic selon la revendication 1, dans lequel ce véhicule comprend une première et une seconde feuille de papier poreux entre lesquelles sont disposés ce support et cet agent d'oxydation.

3. Un agent auxiliaire de diagnostic selon la revendication 1 ou 2, dans lequel ce solvant est un alcool aliphatique inférieur, le chloroforme, le benzène ou un mélange de ceux-ci.

4. Un agent auxiliaire de diagnostic selon la revendication 3 dans lequel ce solvant est un alkanol ou alkénol possédant de 1 à 6 atomes de carbone.

5. Un dispositif selon la revendication 4, dans lequel ce solvant comprend le méthanol, l'isopropanol ou un mélange de ceux-ci.

6. Un agent auxiliaire de diagnostic selon l'une quelconque des revendications 1 à 5, dans lequel ce gaiac est présent dans cette solution en quantité de 1% à 5% en poids de la solution avant d'être adsorbé sur ce support ou absorbé dans celui-ci.

7. Un agent auxiliaire de diagnostic selon la revendication 6, dans lequel ce gaiac est présent dans cette solution en quantité de 1 % à 3 % en poids de la solution.

8. Un agent auxiliaire de diagnostic selon l'une quelconque des revendications 1 à 7, dans lequel le rapport en poids de cet agent d'oxydation à ce support est d'environ 1/3 à environ 3/1.

9. Un agent auxiliaire de diagnostic selon l'une quelconque des revendications 1 à 8, dans lequel cet agent d'oxydation est sous une forme particulaire.

10. Un agent auxiliaire de diagnostic selon la revendication 9, dans lequel cet agent d'oxydation est le persulfate de potassium ou un autre dérivé de monopersulfate, l'hydropéroxyde de cumène, l'hydropéroxyde de butyle tertiaire ou un de leurs mélanges.

11. Un agent auxiliaire de diagnostic selon la revendication 9, dans lequel cet agent d'oxydation est un sel triple de monopersulfate de potassium, de sulfate acide de potassium et sulfate de potassium.

12. Un agent auxiliaire de diagnostic selon l'une quelconque des revendications précédentes dans lequel ce support et cet agent d'oxydation sont tous les deux sous forme particulaire et se trouvent en mélange.

13. Un agent auxiliaire de diagnostic selon l'une quelconque des revendications 1 à 8 dans lequel cet agent d'oxydation est sous forme liquide et est adsorbé sur le support ou absorbé dans celui-ci.

14. Un agent auxiliaire de diagnostic selon la revendication 13, dans lequel cet agent d'oxydation et cette solution de gaiac sont adsorbés sur un support classique ou absorbés dans celui-ci.

15. Un agent auxiliaire de diagnostic selon l'une quelconque des revendications 1 à 8 dans lequel cet agent d'oxydation est en solution dans un de ces solvants, cet agent d'oxydation en solution étant adsorbé sur le support ou absorbé dans celui-ci.

16. Un agent auxiliaire de diagnostic selon la revendication 15, dans lequel cet agent d'oxydation est l'hydropéroxyde de cumène, l'hydropéroxyde de butyle tertiaire, le péroxyde de baryum, le péroxyde de strontium, le péroxyde de magnésium ou un de leurs mélanges.

17. Un agent auxiliaire de diagnostic selon l'une quelconque des revendications 13 à 15 dans lequel cet agent d'oxydation est le péroxyde d'hydrogène.

18. Procédé de détermination de la présence d'hémoglobines dans un milieu aqueux, caractérisé en ce qu'il consiste à mettre le milieu aqueux en contact avec un agent auxiliaire de diagnostic selon l'une quelconque des revendications 1 à 17 et à déterminer si une portion du support de cet agent auxiliaire de diagnostic est colorée en bleu.


**Patentansprüche**

1. Ein im wesentlichen trockenes diagnostisches Hilfsmittel, geeignet zur Verwendung in einer einstufigen Diagnose mit einem Adsorptionsmittel- oder Absorptionsmittelträger darauf oder darin, welcher eine Lösung von Guajak in einem Lösungsmittel dafür adsorbiert oder absorbiert, und in Kontakt mit dem Träger mit einem Oxidationsmittel, das in der Lage ist, das Guajak zu einem blauen Farbstoff in Gegenwart von Wasser und Hämoglobin zu oxidieren, wobei das Oxidationsmittel in Abwesenheit von Wasser im wesentlichen nicht-reaktiv mit dem Guajak ist, wenn es auf dem bzw. in den Träger adsorbiert oder obsorbiert ist, wobei ein wasserdurchlässiges Vehikel, das den Träger und das Oxidationsmittel hält, vorgesehen ist, dadurch gekennzeichnet, daß der in teilchenförmiger Form vorliegende Träger aus einem Material auf Siliciumoxidbasis, Calciumphosphat und Gemischen davon ausgewählt ist und das Guajak hält, um leicht verfügbar in Gegenwart von Hämoglobin und dem Oxidationsmittel in einer wässrigen Umgebung zu reagieren, um den blauen Farbstoff zu bilden.

2. Diagnostisches Hilfsmittel nach Anspruch 1, wobei das Vehikel eine erste und eine zweite Lage eines porösen Papiers umfaßt, zwischen denen der Träger und das Oxidationsmittel angeordnet sind.

3. Diagnostisches Hilfsmittel nach Anspruch 1 oder 2, wobei das Lösungsmittel ein niedriger aliphatischer Alkohol, Chloroform, Benzol oder ein Gemisch davon ist.

4. Diagnostisches Hilfsmittel nach Anspruch 3, wobei das Lösungsmittel ein Alkanol oder Alkenol mit 1 bis 6 Kohlenstoffatomen ist.

5. Diagnostisches Hilfsmittel nach Anspruch 4, wobei das Lösungsmittel Methanol, Isopropanol oder ein Gemisch davon umfaßt.

6. Diagnostisches Hilfsmittel nach einem der Ansprüche 1 bis 5, wobei das Guajak in dieser Lösung in einer Menge von 1 % bis 5 %, bezogen auf das Gewicht der Lösung, vorliegt, bevor es auf dem bzw. in den Träger adsorbiert bzw. absorbiert wird.

7. Diagnostisches Hilfsmittel nach Anspruch 6, wobei das Guajak in dieser Lösung in einer Menge von 1 % bis 3 %, bezogen auf das Gewicht der Lösung, vorliegt.

8. Diagnostisches Hilfsmittel nach einem der Ansprüche 1 bis 7, wobei das Gew.-Verhältnis des Oxidationsmittels zu dem Träger etwa 1 : 3 bis etwa 3 : 1 beträgt.

9. Diagnostisches Hilfsmittel nach einem der Ansprüche 1 bis 8, wobei das Oxidationsmittel teilchenförmig ist.

10. Diagnostisches Hilfsmittel nach Anspruch 9, wobei das Oxidationsmittel Kaliumpersulfat oder eine andere Monopersulfatverbindung, Cumolhydroperoxid, tertiäres Butyl-hydroperoxid oder ein Gemisch davon ist.

11. Diagnostisches Hilfsmittel nach Anspruch 9, wobei das Oxidationsmittel ein dreifaches Salz von Kaliummonopersulfat, Kaliumhydrogensulfat und Kaliumsulfat ist.

12. Diagnostisches Hilfsmittel nach einem der vorstehenden Ansprüche, wobei der Träger und das Oxidationsmittel beide teilchenförmig und in einem Gemisch vorliegen.

13. Diagnostisches Hilfsmittel nach einem der Ansprüche 1 bis 8, wobei das Oxidationsmittel in flüssiger Form vorliegt und auf dem oder in den Träger adsorbiert bzw. absorbiert ist.

14. Diagnostisches Hilfsmittel nach Anspruch 13, wobei das Oxidationsmittel und die Guajaklösung auf einen oder in einem Träger adsorbiert bzw. absorbiert sind.

15. Diagnostisches Hilfsmittel nach einem der Ansprüche 1 bis 8, wobei das Oxidationsmittel in Lösung in einem Lösungsmittel dafür vorliegt und das Oxidationsmittel in Lösung auf dem oder in den Träger adsorbiert bzw. absorbiert ist.

16. Diagnostisches Hilfsmittel nach Anspruch 15, wobei das Oxidationsmittel Cumol-hydroperoxid, tertiäres Butyl-hydroperoxid, Bariumperoxid, Strontiumperoxid, Magnesiumperoxid oder ein Gemisch davon ist.

17. Diagnostisches Hilfsmittel nach einem der Ansprüche 13 bis 15, wobei das Oxidationsmittel Wasserstoffperoxid ist.

18. Verfahren zur Bestimmung der Gegenwart von Hämoglobin in einem Wässrigen Medium, dadurch gekennzeichnet, daß es das Inberührungbringen des wässrigen Mediums mit einem diagnostischen Hilfsmittel nach einem der Ansprüche 1 bis 17 und die Bestimmung, bo ein Teil des Trägers des diagnostischen Hilfsmittels sich blau verfärbt, umfaßt.